# EUROPEAN PATENT SPECIFICATION

(11) **EP 3 613 425 B1**
(45) Date of publication and mention of the grant of the patent: **18.10.2023**
(21) Application number: 18781817.4
(22) Date of filing: 03.04.2018
(51) Int. Cl.: A23L 33/105, A61K 36/185, A61K 36/21, A61K 36/45, A61K 36/74, A61K 36/82, A61K 36/87, A61P 3/04, A61P 3/10, A61P 5/24, A61P 9/00, A61P 15/00, A61P 17/16, A61Q 17/00, A61Q 19/08, A61K 9/08, A61K 9/48, A61K 36/61, A61K 36/752

(54) **PLANT EXTRACT COMPOSITION PROMOTING COLLAGEN GROWTH, REDUCING WRINKLES AND ENHANCING SKIN AND JOINT ELASTICITY**
PFLANZENEXTRAKTZUSAMMENSETZUNG ZUR FÖRDERUNG DES KOLLAGENWACHSTUMS, ZUR VERMINDERUNG VON FALTEN UND VERBESSERUNG DER HAUT- UND GELENKELASTIZITÄT
COMPOSITION PHARMACEUTIQUE D'EXTRAIT DE PLANTES STIMULANT LA PRODUCTION DE COLLAGÈNE POUR RÉDUIRE LES RIDES ET AMÉLIORER L'ÉLASTICITÉ DE LA PEAU ET DES ARTICULATIONS

(30) Priority: 03.04.2017 US 201762480860 P; 08.05.2017 US 201762503185 P
(43) Date of publication of application: 26.02.2020
(73) Proprietor: TCI Co., Ltd., Taipei 11494 (TW)
(72) Inventor: LIN, Yung-Hsiang, Taipei City 11494 (TW); CHEN, I-Hui, Taipei City 11494 (TW); KAN, Kai-Wen, Taipei City 11494 (TW); LIU, Fu Chen, Taipei City 11494 (TW); CHEN, Ciao-Ting, Taipei City 11494 (TW)
(74) Representative: Viering, Jentschura & Partner mbB Patent- und Rechtsanwälte
(86) International application number: PCT/CN2018/081716
(87) International publication number: WO 2018/184524

(56) References cited:
- CN-A- 1 173 991
- CN-A- 102 613 572
- CN-A- 102 763 885
- CN-A- 104 543 983
- CN-A- 104 783 288
- CN-A- 105 194 102
- CN-A- 105 325 608
- CN-A- 105 494 827
- CN-A- 105 639 355
- CN-A- 106 455 637
- CN-A- 106 492 084
- CN-B- 102 423 374
- KR-A- 20060 064 038
- KR-A- 20070 106 175
- KR-A- 20150 052 473
- US-A1- 2005 048 008
- US-A1- 2006 251 608
- US-A1- 2015 238 411
- DATABASE GNPD [Online] MINTEL; 1 November 2011 (2011-11-01), anonymous: "Eye Creme", XP055543121, retrieved from www.gnpd.com Database accession no. 1671722
- YUMI AOKI ET AL: "Melanogenesis Inhibition by an Oolong Tea Extract in B16 Mouse Melanoma Cells and UV-Induced Skin Pigmentation in Brownish Guinea Pigs", BIOSCIENCE, BIOTECHNOLOGY, AND BIOCHEMISTRY, vol. 71, no. 8, 23 August 2007 (2007-08-23), pages 1879-1885, XP055132753, ISSN: 0916-8451, DOI: 10.1271/bbb.70099
- DATABASE GNPD [Online] MINTEL; 31 March 2017 (2017-03-31), anonymous: "Kale + Aloe Sunflower Oil Tripeptide 5 Age Prevention Superfood Eye Cream", XP055732079, retrieved from www.gnpd.com Database accession no. 4635683
- SHU UEMURA: ""Phyto Black Lift Skincare" revitalizing firming bio-cellulose mask", GNPD, MINTEL, 1 November 2010 (2010-11-01), XP002664778,
- DATABASE GNPD [Online] MINTEL; 8 September 2016 (2016-09-08), anonymous: "Berry Fusion Dietary Supplement", XP055732092, retrieved from www.gnpd.com Database accession no. 4266801
- DATABASE GNPD [Online] MINTEL; 20 April 2017 (2017-04-20), anonymous: "Multi-Tasking Neck Cream", XP055732101, retrieved from www.gnpd.com Database accession no. 4661513
- DATABASE GNPD [Online] MINTEL; 22 January 2004 (2004-01-22), anonymous: "Blueberry Extract (Supplement Gum)", XP055732112, retrieved from www.gnpd.com Database accession no. 247570
- Anonymous: "Pu-erh Tea Nourishing Cream | Lizora", , 14 August 2016 (2016-08-14), XP055732106, Retrieved from the Internet: URL:https://www.lizora.com/products/pu-erh -tea-nourishing-cream [retrieved on 2020-09-18]
- WANG Liuxiang: "Protective effect of EGCG against oxidative damages induced by UVA in human skin fibroblast", Master Thesis, no. 02, 15 February 2014 (2014-02-15), pages 1-87, XP009516639,
- XU Qingping et al.: "Antioxydant activity of Spinach extracts", Modern Food Science and Technology, vol. 23, no. 2, 15 February 2007 (2007-02-15), pages 31-32-36, XP009516903, DOI: 10.13982/j.mfst.1673-9078.2007.02.010
- BAE, J. Y.: "Bog blueberry anthocyanins alleviate photoaging in ultravio- let-B irradiation-induced human dermal fibroblasts", Molecular Nutrition & Food Research, vol. 53, no. 6, 9 June 2009 (2009-06-09), pages 726-738, XP055612763,
- MARIA DEL CARMEN VELAZQUEZ PEREDA et al.: "Effect of green Coffea Arabica L. seed oil on extracellular matrix components and water-channel expression in in vitro and ex vivo human skin models", Journal of Cosmetic Dermatology, vol. 8, no. 1, 17 February 2009 (2009-02-17), - March 2009 (2009-03), pages 56-62, XP055612766,
- LIU Xue-ren: "Advances in Studies on the Biological Activities of Hesperidin and Hesperetin", Chinese Journal and New Drugs, vol. 20, no. 4, 31 December 2011 (2011-12-31), pages 329-333-381, XP009516923, ISSN: 1003-3734
- Apraj Vinita D ET AL: "Evaluation of Skin Anti-aging Potential of Citrus reticulata Blanco Peel Correspondence", Jul-Sep, 1 January 2016 (2016-01-01), pages 160-168, XP055775655, Retrieved from the Internet: URL:https://www.phcogres.com/temp/PhcogRes 83160-7152172_195201.pdf [retrieved on 2021-02-12]
- DATABASE GNPD [Online] MINTEL; 26 January 2017 (2017-01-26), anonymous: "C + E Antioxidant Protect + Repair Moisturizer", XP055818903, Database accession no. 4552279
- Anonymous: "Basilur Tea Four Seasons Spring Tea (Karton) | Grüner Tee | Teapur", teapur.de, 31 May 2016 (2016-05-31), XP055892056, Retrieved from the Internet: URL:https://www.teapur.de/gruener-tee/596/ basilur-tea-four-seasons-spring-tea-karton -online-kaufen [retrieved on 2022-02-15]
- DATABASE GNPD [Online] MINTEL; 20 March 2017 (2017-03-20), anonymous: "Ultimate Protection UV Moisturizer SPF 33 PA+++", XP055970439, Database accession no. 4580643

## Description

### CROSS-REFERENCE TO RELATED APPLICATION

This application claims priority of US application No. 62/480,860, filed on April 3, 2017 and US application No. 62/503,185, filed on May 8, 2017.

### BACKGROUND OF THE INVENTION

### 1. Field of the Invention

The present invention relates to a composition containing plant extracts and the use thereof, and more particularly to the composition containing plant extracts and the use thereof for enhancing the synthesis of collagen.

### 2. The Prior Art

Collagen is the most important structural protein in animals and the main component of extracellular matrix and connective tissue. Collagen is widely found in tissues such as tendons, bones, skin and cornea and is the most abundant protein in animals which accounts for 20-30% of the total protein content and 6% of the total body weight of the animal.

In the case of sufficient protein nutrition and vitamin C, collagen can be synthesized by the body itself. However, the synthesis of collagen decreases and the loss rate of collagen increases with age, and problems of loss of collagen would happen such as loose skin or joint wear.

Therefore, if the amount of collagen secretion in the body can be properly increased, the skin can be kept elastic, the formation of wrinkles can be slowed down, the strength and the flexibility of joints can be increased, and the wear and aging of joints can be slowed down. However, the ingredients or drugs currently available for promoting the increase in the amount of collagen secretion are limited. If a certain plant extract is used to promote the increase in the amount of collagen secretion, it must be administered at a relatively high dose, and thus there are limitations in the cost of use and effect. Therefore, if a composition containing plurality of plant extracts with a small amount of each component can be administered to produce the similar effect when administered a high-dose of a single component, the use of the aforementioned effects can be greatly improved, and at the same time, the use cost can be reduced.

### SUMMARY OF THE INVENTION

According to the invention, a composition according to claim 1 is provided. Some examples of such a composition are defined in dependent claims 2 to 4. Moreover, a non-therapeutic method is defined in claim 5. An example of such a method is defined in dependent claim 6.

To solve the foregoing problem, one objective of the present invention is to provide a composition comprising a plant extract, wherein the plant extract comprises at least one combination selected from the group consisting of a red wine extract and a blueberry extract (not covered by the present invention), a citrus extract and a blueberry extract (not covered by the present invention), a citrus extract and a Four Seasons Spring tea extract (the present invention), a spinach extract and a black tea extract (not covered by the present invention), a spinach extract and a green tea extract (not covered by the present invention), a green coffee bean extract and a blueberry extract (not covered by the present invention), and a green coffee bean extract and a Pu-erh tea extract (not covered by the present invention).

In one embodiment of the present invention, the composition comprises any of the following combinations: at least 0.003906 mg/mL of the green coffee bean extract and at least 0.003906 mg/mL of the blueberry extract (not covered by the present invention); at least 0.015625 mg/mL of the green coffee bean extract and at least 0.015625 mg/mL of the Pu-erh tea extract (not covered by the present invention); at least 0.125 mg/mL of the blueberry extract and at least 0.125 mg/mL of the red wine extract (not covered by the present invention); at least 0.25 mg/mL of the spinach extract and at least 0.25 mg/mL of the green tea extract (not covered by the present invention); at least 0.5 mg/mL of the spinach extract and at least 0.5 mg/mL of the black tea extract (not covered by the present invention); at least 0.5 mg/mL of the citrus extract and at least 0.5 mg/mL of the Four Seasons Spring tea extract (the present invention), and at least 4 mg/mL of the citrus extract and at least 4 mg/mL of the blueberry extract (not covered by the present invention).

Disclosed is a pharmaceutical composition for enhancing the synthesis of collagen, comprising the aforementioned composition and a pharmaceutical acceptable carrier.

The pharmaceutical composition may be in a form of a solution, a capsule or a tablet.

An objective of the present invention is to provide a food composition for enhancing the synthesis of collagen, comprising the aforementioned composition and a food ingredient, wherein the food ingredient is a component of a general food, a health food, a dietary supplement, or a drink.

The references to methods of treatments in the subsequent paragraphs of this description are to be interpreted as references to the compounds, pharmaceutical compositions and medicaments of the present invention for use in a method for treatment of the human (or animal) body by therapy (or for diagnosis).

Another objective of the present invention is to provide a method for enhancing the synthesis of collagen, comprising administering to a subject in need thereof an effective amount of the aforementioned composition, wherein the composition is a food product.

The composition comprising plant extracts of the present invention can effectively promote the synthesis of collagen, effectively enhance the synthesis rate of collagen, enhance the elasticity of the skin, reduce the wrinkle formation, and improve the strength and flexibility of the joint.

### BRIEF DESCRIPTION OF THE DRAWINGS

The following drawings form part of the present specification and are included here to further demonstrate some aspects of the present invention, which can be better understood by reference to one or more of these drawings, in combination with the detailed description of the embodiments presented herein.

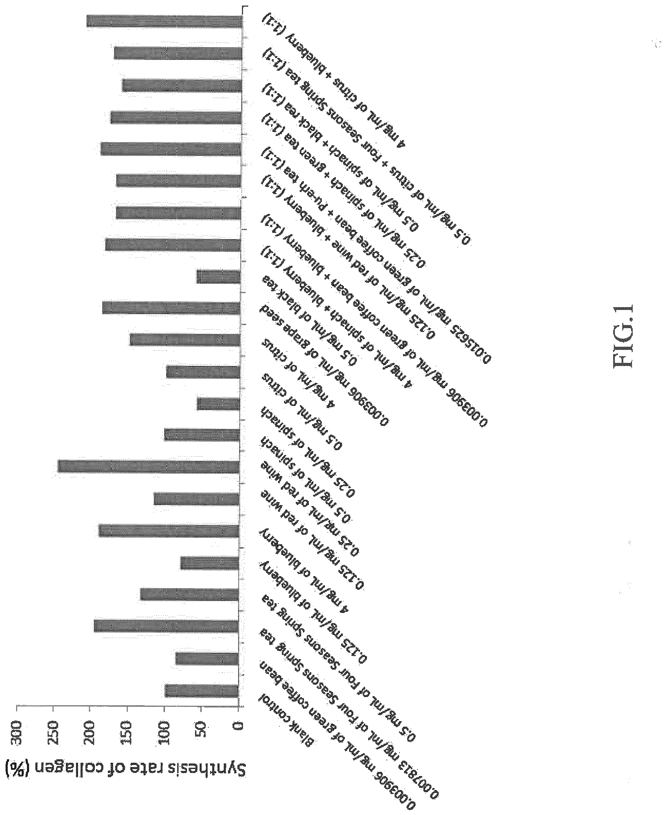

FIG. 1 is a diagram showing the effects of different plant extracts or combinations on the enhancement of synthesis rate of collagen.

### DETAILED DESCRIPTION OF THE PREFERRED EMBODIMENT

The embodiments of the present invention are further described with the following drawings. The following embodiments are given to illustrate the present invention and are not intended to limit the scope of the present invention.

Therefore, the scope of the present invention is defined by the scope of the appended claims.

### Definition

As used herein, the data provided represent experimental values that can vary within a range of ±20%, preferably within ±10%, and most preferably within ±5%.

### Example 1 Preparation of each plant extract

### 1-1 Preparation of Four Seasons Spring tea extract (the present invention)

The Four Seasons Spring tea extract was prepared as follows: first, the leaves of Four Seasons Spring tea plant were washed and dried, and then Four Seasons Spring tea were coarsely crushed by a pulverizer to be a crude material. The crude material of Four Seasons Spring tea was extracted using water as the solvent. The weight ratio of the solvent to the crude material was 5-20:1-5. The extraction temperature was at 50-100°C, preferably 75-95°C, for 0.5-3 hours to obtain the crude extract of the Four Seasons Spring tea. After the extraction, the crude extraction was cooled to room temperature. The crude extraction was filtered through a 400 mesh filter to remove residual solids. Furthermore, the filtered of the Four Seasons Spring tea was further concentrated under reduced pressure at 45-70°C to obtain a concentrated product and the concentrated product was the Four Seasons Spring tea extract of the present invention.

### 1-2 Preparation of green tea extract (not covered by the present invention)

The green tea extract was prepared as follows: first, the unfermented leaves of *Camellia sinensis,* i.e. the green tea, were washed and dried, and then were coarsely crushed by a pulverizer to be a crude material. The crude material of green tea was extracted using water as the solvent. The weight ratio of the solvent to the crude material was 5-20:1-5. The extraction temperature was at 50-100°C, preferably 75-95°C, for 0.5-3 hours to obtain the crude extract of the green tea. After the extraction, the crude extraction was cooled to room temperature. The crude extraction was filtered through a 400 mesh filter to remove residual solids. Furthermore, the filtered of the green tea was further concentrated under reduced pressure at 45-70°C to obtain a concentrated product and the concentrated product was the green tea extract of the present invention.

### 1-3 Preparation of black tea extract (not covered by the present invention)

The black tea extract was prepared as follows: first, the fermented leaves of *Camellia sinensis,* i.e. the black tea, were washed and dried, and then were coarsely crushed by a pulverizer to be a crude material. The crude material of black tea was extracted using water as the solvent. The weight ratio of the solvent to the crude material was 5-20:1-5. The extraction temperature was at 50-100°C, preferably 75-95°C, for 0.5-3 hours to obtain the crude extract of the black tea. After the extraction, the crude extraction was cooled to room temperature. The crude extraction was filtered through a 400 mesh filter to remove residual solids. Furthermore, the filtered of the black tea was further concentrated under reduced pressure at 45-70°C to obtain a concentrated product and the concentrated product was the black tea extract of the present invention.

### 1-4 Preparation of spinach extract (not covered by the present invention)

The spinach extract was obtained by extracting spinach (*Spinacia oleracea*), and the spinach extract used in the example was purchased from Honh Siang Farm Products Factory.

### 1-5 Preparation of red wine extract (not covered by the present invention)

The red wine extract was obtained by extracting red wine, and the red wine extract used in the example was purchased from Shanghai Boyoutang Biotechnology Co., Ltd.

### 1-6 Preparation of citrus extract (the present invention)

The citrus extract was obtained by extracting the fruits of *Citrus reticulata,* and the citrus extract used in the example was purchased from Roterm Trading Co., Ltd.

### 1-7 Preparation of green coffee bean extract (not covered by the present invention)

The green coffee bean extract was obtained by extracting the seeds of unroasted coffee (*Coffea spp.*), and the green coffee bean extract used in the example was purchased from Arjuna Natural Extracts Ltd.

### 1-8 Preparation of blueberry extract (not covered by the present invention)

The blueberry extract was obtained by extracting the fruits of *Vaccinium Cyanococcus,* and the blueberry extract used in the example was purchased from BIOMED HERBAL RESEARCH CO., LTD.

### 1-9 Preparation of Pu-erh tea extract (not covered by the present invention)

The Pu-erh tea extract was obtained by extracting the post-fermented leaves of *Camellia sinensis,* and the Pu-erh tea extract used in the example was purchased from Nanjing Zelang Biotechnology Co., Ltd.

### 1-10 Preparation of grape seed extract (not covered by the present invention)

The grape seed extract was purchased from Guarante Biotech Co., Ltd.

### Example 2 Effects of combination of the plant extracts on enhancing the synthesis of collagen in fibroblast cells

The present invention analyzed the synthesis of collagen by human skin fibroblast (CCD-966sk). The human skin fibroblast was purchased from the Food Industry Research and Development Institute, Taiwan, and the number is BCRC 60153. The cells were cultured in the culture medium with 10% fetal bovine serum and 90% minimum essential medium (MEM, purchased from Gibco, USA) and the medium further contains 1 mM sodium pyruvate and 1% penicillin or streptomycin.

First, 2×10⁴ of the human skin fibroblasts were seeded in 24-well culture plates containing 500 µL of the above culture medium, and were cultured at 37°C. After 24 hours culturing, the cells were washed by phosphate buffered saline (PBS) without disturbing the cells, and then the cells were divided into the following 21 groups and added to 500 µL of the MEM without any fetal bovine serum: (1) 0.003906 mg/mL of the green coffee bean extract, (2) 0.007813 mg/mL of the Four Seasons Spring tea extract, (3) 0.5 mg/mL of the Four Seasons Spring tea extract, (4) 0.125 mg/mL of the blueberry extract, (5) 4 mg/mL of the blueberry extract, (6) 0.125 mg/mL of the red wine extract, (7) 0.25 mg/mL of the red wine extract, (8) 0.25 mg/mL of the spinach extract, (9) 0.5 mg/mL of the spinach extract, (10) 0.5 mg/mL of the citrus extract, (11) 4 mg/mL of the citrus extract, (12) 0.003906 mg/mL of the grape seed extract, (13) 0.5 mg/mL of the black tea extract, (14) 4 mg/mL of the combination of the spinach extract and the blueberry extract mixed in a ratio of 1:1, (15) 0.003906 mg/mL of the combination of the green coffee bean extract and the blueberry extract mixed in a ratio of 1:1, (16) 0.015625 mg/mL of the combination of the green coffee bean extract and the Pu-erh tea extract mixed in a ratio of 1:1, (17) 0.125 mg/mL of the combination of the blueberry extract and the red wine extract mixed in a ratio of 1:1, (18) 0.25 mg/mL of the combination of the spinach extract and the green tea extract mixed in a ratio of 1:1, (19) 0.5 mg/mL of the combination of the spinach extract and the black tea extract mixed in a ratio of 1:1, (20) 0.5 mg/mL of the combination of the Four Seasons Spring tea extract and the citrus extract mixed in a ratio of 1:1 (the present invention), and (21) 4 mg/mL of the combination of the citrus extract and the blueberry extract mixed in a ratio of 1:1; wherein, the cells treated without any extract was as the blank control group, and the culture medium was as the background for the subsequent reading absorbance. After culturing at 37°C for 48 hours, 1 mL of the culture medium of each group was collected without disturbing the cells, and was then placed in a 1.5 mL eppendorf. The amount of collagen secreted by the skin fibroblasts of each group was detected by the soluble collagen assay kit (SircolTM Soluble Collagen Assay kit, Bicolor Life Science Assays, Notherm Ireland, the UK). First, 200 µL of the collagen isolation & concentration reagent was added into each of the eppendorf and was upside down to mix well. After reacted at 4°C for 16-18 hours, each eppendorf was centrifuged at 12000 rpm for 10 minutes at 4°C, avoiding shaking the tube during operation. Then, 1 mL of the supernatant was carefully removed, and 1 mL of Sircol dye reagent was added into each eppendorf to stain the collagen. After each eppendorf was gently shaken for 30 minutes to mix well, it was then centrifuged at 12000 rpm for 10 minutes at 4°C and the supernatant was removed. Then, 750 µL of Acid-salt washing reagent was added into each eppendorf to wash away the excess dye. After centrifuged at 12000 rpm for 10 minutes at 4°C, the supernatant was removed and the liquid remaining in the wall and lid of the eppendorf was carefully removed with a cotton ball. Then, 250 µL of Alkali reagent was added into each eppendorf and the collagen was uniformly dissolved by a vortex. Finally, the absorbance of the product of the 21 groups and the blank control group at 555 nm was measured by an enzyme linked immunosorbent assay, ELISA, analyzer. After deducting the background, the absorbance of the differences between the 21 groups and the blank control group was calculated, and the student *t-test* was used to determine the standard deviation and the statistically significant difference (* p< 0.05; ** p<0.01; *** p<0.001).

The results of each group of the combination of the plant extract of the present invention enhancing the synthesis rate of collagen was shown in Table1 and FIG.1; wherein, the higher the percentage, the better the ability to enhance the synthesis of collagen. As showing in Table1 and FIG.1, compared with the blank control group, the green coffee bean extract, 0.125 mg/mL of the blueberry extract, the spinach extract, 0.5 mg/mL of the citrus extract, and 0.5 mg/mL of the black tea extract could not significantly enhance the synthesis of collagen, and some of them even significantly reduced the synthesis of collagen, such as the green coffee bean extract, 0.125 mg/mL of the blueberry extract, 0.25 mg/mL of the spinach extract, 0.5 mg/mL of the citrus extract, and 0.5 mg/mL of the black tea extract (see the comparative groups). However, except the combination of the spinach extract and the blueberry extract, the synthesis rate of collagen in the groups of the combination of the green coffee bean extract and the blueberry extract, the combination of the green coffee bean extract and the Pu-erh tea extract, the combination of the red wine extract and the blueberry extract, the combination of the spinach extract and the green tea extract, the combination of the spinach extract and the black tea extract, the combination of the citrus extract and the Four Seasons Spring tea extract (the present invention), and the combination of the citrus extract and the blueberry extract (see the experimental groups) were all enhanced much more than that in each group of single plant extract with same concentration. The result indicates that the specific combinations described above unexpectedly exhibit a higher ability to enhance the synthesis of collagen than a single plant extract, and not a random combination can achieve such effect.

**Table 1 The synthesis rate of collagen of each group**

| Group | Plant extract/ Combination | Concentration (mg/mL)/Ratio | Synthesis rate of collagen (%) |
|---|---|---|---|
| Control | - | - | 100 |
| Comparative group 1 | green coffee bean | 0.003906 | 85.46 |
| Comparative group 2 | Four Seasons Spring tea | 0.007813 | 195.14 |
| Comparative group 3 | | 0.5 | 132.45 |
| Comparative group 4 | blueberry | 0.125 | 78.26 |
| Comparative group 5 | | 4 | 189.46 |
| Comparative group 6 | red wine | 0.125 | 116 |
| Comparative group 7 | | 0.25 | 246.29 |
| Comparative group 8 | spinach | 0.25 | 57.82 |
| Comparative group 9 | | 0.5 | 102.12 |
| Comparative group 10 | citrus | 0.5 | 99.24 |
| Comparative group 11 | | 4 | 148.92 |
| Comparative group 12 | grape seed | 0.003906 | 186.27 |
| Comparative group 13 | black tea | 0.5 | 59.39 |
| Comparative group 14 | spinach + blueberry | 4 (1:1) | 182.92 |
| Experiment group 1 | green coffee bean + blueberry | 0.003906 (1:1) | 168.33 |
| Experiment group 2 | green coffee bean + Pu-erh tea | 0.015625 (1:1) | 168.03 |
| Experiment group 3 | red wine + blueberry | 0.125 (1:1) | 189.46 |
| Experiment group 4 | spinach + green tea | 0.25 (1:1) | 176.96 |
| Experiment group 5 | spinach + black tea | 0.5 (1:1) | 161.71 |
| Experiment group 6 | citrus + Four Seasons Spring tea (the present invention) | 0.5 (1:1) | 172.85 |
| Experiment group 7 | citrus + blueberry | 4 (1:1) | 210.34 |

In summary, the compositions of the present invention significantly enhance the synthesis collagen by mixing the specific plant extracts or mixing the specific plant extracts with the plant-derived compounds. Therefore, the composition can be utilized to prepare pharmaceutical or food compositions that enable to enhance the elasticity of the skin, reduce the wrinkle formation, and improve the strength and flexibility of the joint of the applicator or consumer.

Furthermore, the composition can be further added to a food, a health food, a dietary supplement, or a drink.

## Claims

1. A composition, comprising a plant extract, wherein the plant extract comprises at least 0.5 mg/mL of a citrus extract and at least 0.5 mg/mL of a Four Seasons Spring tea extract, the Four Seasons Spring tea extract is obtained by water extraction, and the weight ratio of water to Four Seasons Spring tea for the extraction falls within a range of 5-20: 1-5.

2. The composition according to claim 1, further comprising a pharmaceutical acceptable carrier.

3. The composition according to claim 2, wherein the composition is in a form of a solution, a capsule, or a tablet,

4. The composition according to claim 1, further comprising a food ingredient, wherein the food ingredient is a component of a general food, a health food, a dietary supplement, or a drink.

5. A non-therapeutic method of enhancing skin elasticity, reducing wrinkle formation, or improving joint strength and flexibility, comprising administering to a subject in need thereof an effective amount of the composition according to claim 1.

6. The non-therapeutic method according to claim 5, wherein the composition is a food product.

## Patentansprüche

1. Eine Zusammensetzung, aufweisend ein Pflanzenextrakt, wobei das Pflanzenextrakt mindestens 0,5 mg/ml eines Zitrusgewächsextrakts und mindestens 0,5 mg/ml eines Vier-Jahreszeiten-Frühling-Tee-Extrakts aufweist, das Vier-Jahreszeiten-Frühling-Tee-Extrakt durch Wasserextraktion erlangt wird und das Gewichtsverhältnis von Wasser zu Vier-Jahreszeiten-Frühling-Tee für die Extraktion in einen Bereich von 5-20:1-5 fällt.

2. Die Zusammensetzung nach Anspruch 1, ferner aufweisend einen pharmazeutisch akzeptablen Träger.

3. Die Zusammensetzung nach Anspruch 2, wobei die Zusammensetzung in Form einer Lösung, einer Kapsel oder einer Tablette vorliegt,

4. Die Zusammensetzung nach Anspruch 1, ferner aufweisend einen Lebensmittelbestandteil, wobei der Lebensmittelbestandteil ein Bestandteil eines allgemeinen Lebensmittelmittels, von Reformkost, eines Nahrungsergänzungsmittels oder eines Getränks ist.

5. Ein nicht-therapeutisches Verfahren zur Verbesserung der Hautelastizität, zur Verringerung der Faltenbildung oder zur Verbesserung der Gelenkfestigkeit und - flexibilität, aufweisend die Verabreichung einer wirksamen Menge der Zusammensetzung nach Anspruch 1 an eine Person, die diese benötigt.

6. Das nicht-therapeutische Verfahren nach Anspruch 5, wobei es sich bei der Zusammensetzung um ein Lebensmittelprodukt handelt.

## Revendications

1. Composition, comprenant un extrait de plante, dans laquelle l'extrait de plante comprend au moins 0,5 mg/mL d'un extrait d'agrumes et au moins 0,5 mg/mL d'un extrait de thé Four Seasons Spring, l'extrait de thé Four Seasons Spring est obtenu par extraction d'eau, et le rapport en poids entre l'eau et le thé Four Seasons Spring pour l'extraction se situe dans une plage de 5-20:1-5.

2. Composition selon la revendication 1, comprenant en outre un support pharmaceutiquement acceptable.

3. Composition selon la revendication 2, dans laquelle la composition est sous la forme d'une solution, d'une capsule, ou d'un comprimé,

4. Composition selon la revendication 1, comprenant en outre un ingrédient alimentaire, dans laquelle l'ingrédient alimentaire est un composant d'une alimentation générale, d'une alimentation diététique, d'un complément alimentaire, ou d'une boisson.

5. Procédé non-thérapeutique pour améliorer l'élasticité de la peau, réduire la formation de rides, ou améliorer la force et la flexibilité d'articulations, comprenant l'administration à un sujet qui en a besoin d'une quantité efficace de la composition selon la revendication 1.

6. Procédé non-thérapeutique selon la revendication 5, dans lequel la composition est un produit alimentaire.
